# EUROPÄISCHE PATENTSCHRIFT

(11) **EP 0 418 420 B1**
(45) Veröffentlichungstag und Bekanntmachung des Hinweises auf die Patenterteilung: **28.02.1996**
(21) Anmeldenummer: 89117552.3
(22) Anmeldetag: 22.09.1989
(51) Int. Cl.: A61F 2/76

(54) **Verfahren zur Herstellung einer Oberschenkelprothese**
Process for the production of an above-knee prosthesis
Procédé pour la fabrication d'une prothèse pour amputé fémoral

(43) Veröffentlichungstag der Anmeldung: 27.03.1991
(73) Patentinhaber: IPOS GmbH & CO. KG., D-21337 Lüneburg (DE)
(72) Erfinder: Prahl, Jan, Ing., D-2127 Rullstorf (DE)
(74) Vertreter: Richter, Werdermann & Gerbaulet

(56) Entgegenhaltungen:
- EP-A- 0 255 797
- FR-A- 1 295 010
- GB-A- 2 080 114
- PROSTHETICS AND ORTHOTICS INTERNATIONAL, Band 9, 1985, Seiten 17-22; C.G. SAUNDERS et al.: "Computer aided desing of prosthetic sockets for below-knee amputees"
- COMPUTER SYSTEMS, Band 6, Nr. 10, Oktober 1986, Seiten 15-16, Bromley, GB; "Shape a leg"

## Beschreibung

Die Erfindung betrifft ein Verfahren zur Herstellung einer Oberschenkelprothese, bei dem aus einem den Restkörperteilmaßen entsprechenden Positivmodell ein Prothesenschaft mit einem an diesem verankerten Gelenkpaßteil geformt und anschließend der Prothesenschaft mit einer kosmetischen Verkleidung der Prothese überzogen oder umhüllt wird.

Insbesondere Oberschenkelprothesen werden unter Verwendung eines Negativmodells hergestellt, welches durch Abformen des entsprechenden Patientenkörperteils erzeugt wird und eine längliche Grundform aufweist, die mindestens an einem Ende offen ausgebildet ist und deren Innenform der Innenfläche des Körper-Anschlußstükkes der herzustellenden Prothese entspricht.

Nach dem Stand der Technik wird eine erhärtende Masse,insbesondere Gips, in verformbarem Zustand auf das Restkörperteil in einer Länge aufgebracht, die dem beabsichtigten Anlage- und Abstützbereich der Prothese an dem Restkörperteil entspricht, wobei das Negativmodell nach dem Erhärten in einer im wesentlichen kuppenförmigen Grundform von dem Restkörperteil abgenommen wird. Nach dem Erhärten wird die Gipsschicht abgenommen und dient als Negativmodell für die Herstellung des Körper-Anschlußstückes der Prothese. Zur eigentlichen Herstellung der Prothese wird entweder ein Positivmodell durch Ausgießen des Negativmodells erzeugt, das für die Erzeugung des Anschlußstückes der Prothese verwendet wird, oder das Anschlußstück muß von Hand hergestellt werden, wobei das Negativmodell als Vorlage dient. Die Fertigung von Hand erfordert eine exakte handwerkliche Arbeit, die relativ viele Fehlermöglichkeiten in sich birgt. Aber auch die Herstellung eines Positivmodells ist nicht problemlos, Verarbeitungsfehler führen dazu, daß das hergestellte Positivmodell letztendlich nicht mehr dem Restkörperteil entspricht. Zumindest ist es bei dieser Herstellungsart erforderlich, daß der Patient zum Vergleich des Positivmodells mit dem tatsächlichen Restkörperteil zur Verfügung steht.
Die Versorgung von Patienten mit Prothesen ist jedoch dann besonders aufwendig, wenn die Patienten in abgelegenen Orten oder Gegenden versorgt werden sollen, in denen selbst keine Versorgung vorgenommen werden kann, und wenn, dann oftmals nur mit primitiven Prothesen oder Prothesenhilfen, wenn Hunderte von Kilometern entfernt z.B. von einer Landeshauptstadt Versorgungen von Patienten mit Prothesen vorgenommen werden sollen. Die Abnahme der für die herzustellenden Prothesen erforderlichen Maße läßt sich vor Ort häufig überhaupt nicht durchführen, wenn die hierzu erforderlichen Einrichtungen fehlen. Werden Negativmodelle angefertigt, dann müssen diese an den Prothesen-Hersteller gesandt werden, der wiederum nach der Herstellung der Prothese diese an den Patienten schicken mußt. Hierzu wird zumindest sehr viel Zeit benötigt, außerdem entstehen hohe Transportkosten bei vielfach langen Transportwegen.

In der EP 0 256 158 ist ferner als nachteilig angesehen worden, daß eine Änderung des Anschlußstückes der Prothese, wie sie durch die Belastungsveränderungen, die für den Patienten entstehen, erforderlich werden können, nur schwer durchgeführt werden kann. Entweder muß ein komplett neues Anschlußstück erstellt werden, oder aber es erfolgt eine nachträgliche Anpassung, die jedoch in ihrer Qualität an die ursprüngliche Anpassung nicht heranreicht. In der genannten Druckschrift wird zur Beseitigung der geschilderten Nachteile vorgeschlagen, an je einem Ende eine Führungskappe an dem Negativmodell zuzuordnen und dort zu befestigen. Diese Führungskappe nimmt je eine Lagervorrichtung für eine Führungseinrichtung auf, die sich von einer Führungskappe zu derjenigen am gegenüberliegenden Ende erstreckt. An dieser Führungseinrichtung ist eine Abtastvorrichtung verschiebbar, z.B. um die Mittelachse dieser Führungseinrichtung drehbar gelagert, wobei die von der Abtastvorrichtung ermittelten Meßdaten über das Profil des Negativmodells zur Herstellung der Prothese verwendet werden können. Dieses Verfahren setzt jedoch ein fehlerfrei hergestelltes Negativmodell voraus. Etwa bei der Negativmodellherstellung aufgetrenen Ungenauigkeiten werden bei der Prothesenherstellung übernommen.

Durch die EP-A- 255 797 ist ein Verfahren zur Herstellung einer Endoprothese mit individueller Anpassung, unter Heranziehung eines mittels Computertomographie erzeugten, mindestens in datenmäßiger Repräsentation vorhandenen, dreidimensionalen Modells mit folgenden Schritten:
- Erzeugung eines Vergleichsmodells für die entsprechenden Knochenbereiche der anderen - symmetrischen - Körperhälfte der Person und Spiegelung oder
- Erzeugung eines Vergleichsmodells für die entsprechenden Knochenbereiche nach mit einem der Körpergröße und der übrigen Konstitution angepaßten nach statischen Durchschnittswerten aufgenommene statische Durchschnittsmodell des jeweiligen Knochenbereichs,
- Diskrimination der Oberflächen von Modell und Vergleichsmodell durch Vergleich in entsprechender räumlicher Ausrichtung,
- Ermittlung mindestens eines Bereiches, in dem Abweichungen von dem Vergleichsmodell vorhanden sind, welche einen vorgegebenen Längen- oder Volumenbetrag überschreiten,
- Ersetzen der abweichenden Bereiche durch die entsprechenden Bereiche des Vergleichsmodells sowie
- Herstellung der Endoprothese nach dem so erhaltenen Modell
bekannt. Mit einem derartigen Verfahren sollte es mit Mitteln der Computertomografie möglich sein, Modelle herzustellen, die dem Knochen in intaktem Zustand entsprechen. Dabei soll es besonders vorteilhaft sein, daß durch die jeweilige symmetrische Knochenpartie in der Regel ein Vergleichsmodell zur Verfügung steht, welches durch Spiegelung in den gewünschten Modellbereicht überführt werden kann, wobei in der Datenverarbeitung durch eine einfache Richtungsvertauschung der die Oberflächenteile bezeichnenden Vektoren bzw. eine entsprechende Änderung der Adressenzuordnung der die Volumen- bzw. Oberflächenelemente enthaltenden Speicherplätze ein Übergang zu spiegelsymmetrischen grafischen Modelldarstellungen möglich ist. Steht ein derartiges von der anderen Körperhälfte genommenes Modell nicht zur Verfügung, kann auf "Konfektionsmodelle" zurückgegriffen werden. Dabei läßt sich der defekte Bereich durch entsprechende Knochenbereiche eines Modells mit Maßen intakter Knochen ersetzen, die aufgrund statistischer Durchschnittsergmaße gewonnen werden und entsprechend unterschiedlichen Körpergrößen und Konstitutionen gespeichert abrufbar sind. Zwischen den unterschiedlichen Größen, die mit den Konfektionsgrößen für Oberbekleidung vergleichbar sind, soll durch Interpolation jede beliebige Zwischengröße erzeugt werden können.

Bei der Durchführung dieser Verfahren sind Fehlerquellen nicht ausschließbar, da z.B. das Umfangsmaß eines Beinstumpfes oder der Beinstumpfkuppe ebenfalls computertomografisch und nicht mechanisch erfaßt wird.

In "Computer aided design of prosthetic sockets for below knee-amputees", C. G. Saunders et al., Prosthetics and Orthotics International, 1985, Band 9, Seiten 17 bis 22, wird ein computergestütztes Formungssystem zur Anwendung im Prothesenbau beschrieben. Eingesetzt werden Computer-Grafik-Terminals u.dgl.. Unterstützt durch eine Software ist das systematische Modifizieren einer einfachen Gelenkpfanne unter Verwendung von Techniken analog zu denen eines mit Gips arbeitenden Prothesenbauers. Dabei wird eine genaue Vermessung des Beinstumpfes vermittels einer Meßlehre vorgenommen, so daß schon mal die Abnahme dieser Werte zu Meßungenauigkeiten führt.

Der Erfindung liegt die Aufgabe zugrunde, das eingangs genannte Verfahren zur Herstellung einer Oberschenkelprothese dahingehend weiter zu entwickeln, daß die Fehlerquellen beim Maßnehmen des Restkörperteils (Positivstumpfes) möglichst minimiert werden, die Meßgenauigkeit insgesamt verbessert wird, wobei der jeweilige Aufwand des Verfahrens und der Vorrichtung durch weitgehende Automatisierung minimiert werden sollen.

Diese Aufgabe wird durch die im Anspruch 1 angegebenen Verfahrensschritte gelöst.

Erfindungsgemäß wird so vorgegangen, daß von mindestens zwei theoretischen Urformen für Restkörperstümpfe und/oder kosmetische Verkleidungen deren jeweilige radiale Umfangslinien scheibenweise als Wertesets (Länge x, Polarkoordinaten y des Radius, Winkelmaß z) gespeichert werden, daß am Patientenrestkörperstumpf jeweils der Umfang oberhalb der sich verjüngenden Stumpfkuppe und in vorgegebenen Abständen im Bereich der sich verjüngenden Stumpfkuppe und/oder der verbliebenen rechten oder linken Extremität (Bein) sowie die jeweilige Länge der Stumpfkuppe und/oder der Extremität gemessen und mit den gespeicherten Wertesets verglichen wird. Die dem Restkörperstumpf oder der Extremität nächstkommende theoretische Urform wird hierbei ausgewählt, wobei die Wertesets dieser theoretischen Urform zur Formung eines Positivmodells verwendet werden, und wobei der Umfang oberhalb der Stumpfkuppe durch Anpassung eines von mehreren, vorzugsweise von drei Formringen ermittelt wird.

Dieses Verfahren hat den Vorteil, daß durch einmaliges Maßnehmen ein Körperstumpf-Positivmodell hergestellt werden kann, welches weitere Patientenbesuche zwecks Prothesenanpassung überflüssig macht. Entsprechendes gilt hinsichtlich der Ausformung der kosmetischen Verkleidung. Sowohl bei der Herstellung des Positivmodells als auch der kosmetischen Verkleidung werden erstmals der Umfang wie auch die sogenannte biomechanische Form (Gliederstärke, Beinform) berücksichtigt. Im Bereich der Stumpfkuppe des Patienten werden dabei mehrere Maße genommen. Zum einen ist der Umfang oberhalb des Verjüngungsbereiches der Stumpfkuppe festzustellen. Hierzu wird eine Anzahl von Formringen verwendet, aus denen durch Anlegen der passende ermittelt wird. Praktische Erfahrungen haben gezeigt, daß bereits drei Formringe ausreichen, um das für die Prothesenherstellung notwendige Umfangsmaß sicher zu bestimmen. Die Verwendung von Formringen hat den Vorteil, daß ein Vermessen bzw. eine Fehlnotierung eines Maßes weitgehend ausgeschlossen werden kann. Darüber hinaus ist es erforderlich, im Verjüngungsbereich der Stumpfkuppe mehrere Umfangsmaße sowie die Länge zu ermitteln. Die Formringgröße sowie die weiteren Umfangsmaße und die Länge geben bereits einen sicheren Aufschluß darüber, welches der gespeicherten Urmodelle zur Prothesenherstellung verwendet werden kann. Insbesondere für die Prothesenverkleidung ist die biomechanische Form der verbliebenen Restextremität (Bein) von erheblicher Bedeutung. Auch diese biomechanische Form wird durch Speicherung verschiedener Urmodelle berücksichtigt.

Die Zahl der Daten hinsichtlich der Urformen kann dadurch reduziert werden, daß diese bzw. deren Wertesets scheibenweise eingeteilt sind, hinsichtlich der Stumpfkuppen können die Scheibenabstände etwa ab 5 cm sein.

Für die kosmetische Verkleidung der Prothese sind erfahrungsgemäß mindestens drei Wertesets von erheblicher Bedeutung, insbesondere bei einer Unterschenkelprothese sind dies die tatsächlichen Maße an dem verbliebenen Bein im Knöchel-, Waden- und im Kniebereich. Sowohl beim Kuppenstumpf als auch bei der kosmetischen Verkleidung lassen sich alle Zwischenbereiche auf den vorgegebenen wenigen Wertesets durch Interpolation rechnerisch bestimmen.

Die Zahl der Urformen, die zu speichern sind, kann dadurch erheblich minimiert werden, daß eine Urform mehrere verschiedene Längen dadurch berücksichtigt, daß die Abstände der Scheiben, für die Wertesets vorliegen, in der Länge variiert werden, wobei sich aus den Variationen die nächstkommende theoretische Urform für die Stumpfkuppe oder die kosmetische Verkleidung durch Vergleich ergibt. Auch hierbei können vorzugsweise die fehlenden, den jeweiligen Umfangsverlauf bestimmenden Werte zwischen den Scheiben durch Interpolation ermittelt werden.

Ist die jeweils nächstkommende Urform bestimmt, können die Positivmodelle wie die Stumpfkuppe und die kosmetische Verkleidung, vorzugsweise durch Abtragen, insbesondere Drehen oder Fräsen, aus einem Formkörper hergestellt werden. Dieser Formkörper soll nach einer Weiterbildung der Erfindung aus Kunststoff bzw. Hartschaumkunststoff bestehen, der durch Fräsen leicht zu bearbeiten ist.

Die Bearbeitung des Formkörpers wird entsprechend den Maßen im Urmodell scheibenweise durchgeführt, wobei vorzugsweise der Fräser von einem Rechnet gesteuert wird, der gleichzeitig als Speicher und Komparator dient. Die computerunterstützte Herstellung (CAM) ist prinzipiell bekannt und wird beispielsweise in der Druckschrift "Prosthetics and Orthotics International", 1985, 9, Seiten 3-24, behandelt, worauf hiermit Bezug genommen wird. Diese vollautomatische Steuerung eines Fräsers durch eine Steuerungseinrichtung (Rechner) soll hier dergestalt ausgenutzt werden, daß der Rechner verschiedene Formringgrößen, z.B. I-III, und Urformen, z.B. zwei Urformgrößen, in Form von Wertesets speichert. Gibt man dem Rechner die verschiedenen Umfangsmaße der Stumpfkuppe und deren Länge sowie beispielsweise die Umfangsmaße Kniegelenk, Wade, Knöchel, die Beinlänge sowie die biomechanische Form (O-Bein) ein, so kann der Rechner durch Vergleich in einem Komparator die passende Urform, gegebenenfalls durch Streckung oder Stauchung einer gespeicherten Urform, ermitteln. Vor diese Urform einer Stumpfkuppe und/oder der kosmetischen Verkleidung liegen alle erforderlichen Werte wie Umfangslinienverlauf, Länge vor; diese Werte werden als Steuerimpulse an einen Fräser gegeben, der das eingespannte Werkstück, den Formkörper aus Hartschaumkunststoff, abträgt.

Vorteilhafte Ausgestaltungen der Erfindung sind in den Unteransprüchen gekennzeichnet.

Das erfindungsgemäße Verfahren wird anhand von in den Zeichnungen dargestellten Ausführungsbeispielen erläutert. Es zeigen
Fig. 1 eine schematische Darstellung eines gesunden Patientenbeines,
Fig. 2 eine schematische Darstellung einer Oberschenkelstumpfes,
Fig. 3 und 4 jeweilige Ansichten eines Körperbeines bzw. Unterschenkels,
Fig. 5 einen Schuh für eine Prothese,
Fig. 6 und 7 jeweils Beinprothesen in verschiedenen Stellungen,
Fig. 8 eine kosmetische Prothesenverkleidung und
Fig. 9 ein Fließschema zur Herstellung eines Positivmodells eines Prothesenstumpfes.

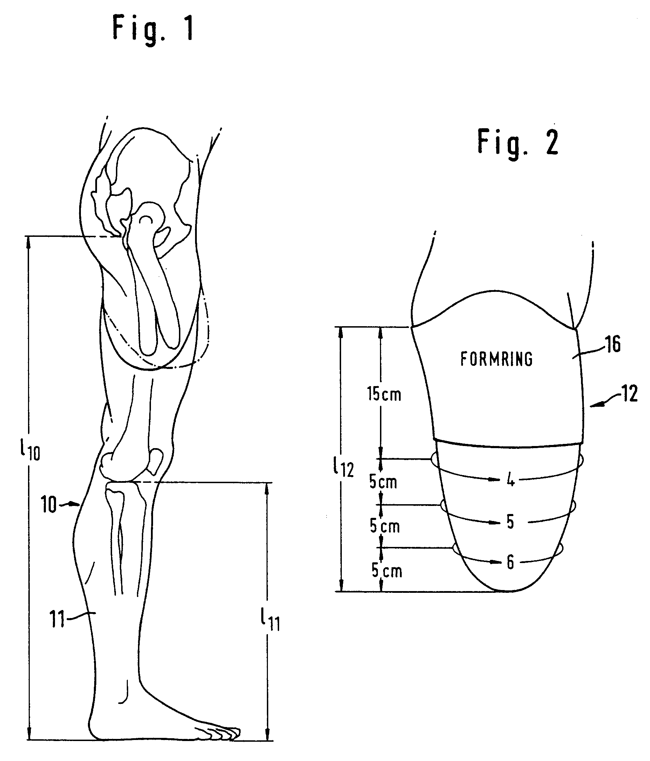

Soll eine Beinprothese angefertigt werden, so sind die Länge, die jeweiligen Umfangsmaße und die biomechanische Form des verbliebenen Patientenbeines zu beachten. In Fig. 1 ist ein Körperbein 10 dargestellt, in dem die Ziffern 1 und 2 jeweilige Oberschenkelbeugungen von 0 bis 5 Grad einerseits und von 5 bis 10 Grad andererseits darstellen. Ziel einer jeden Prothesenherstellung ist es, die Prothese der Beinlänge l₁₀ und der Unterschenkellänge l₁₁ anzupassen.

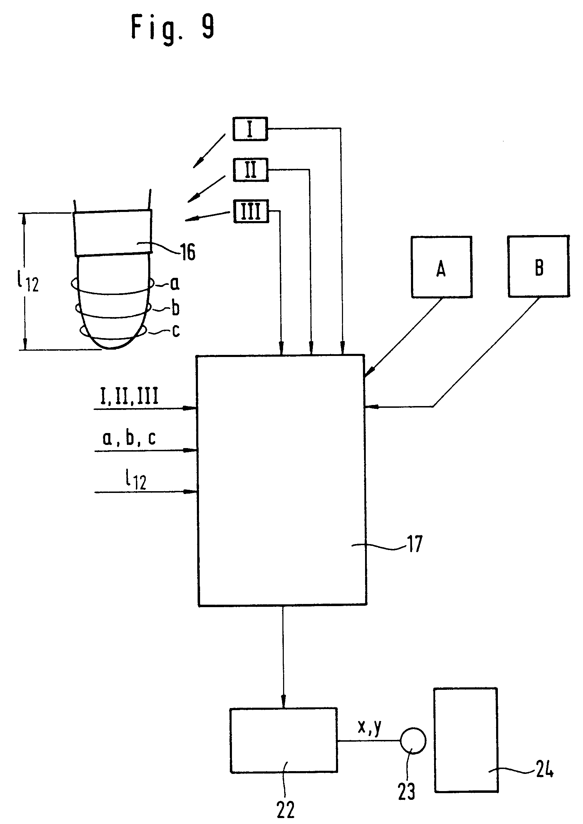

Fig. 2 stellt ein Oberschenkelrestkörperteil 12 dar, das nach einer Oberschenkelamputation die Form einer Stumpfkuppe ist. Diese Stumpfkuppe besitzt neben einem oberen im wesentlichen umfangsgleichen Bereich einen konischen Bereich, dessen Länge und Konizität hinsichtlich des herzustellen Prothesen-Anschlußstückes zu vermessen ist. Hierzu wird zunächst von der Stumpfkuppe 12 das Umfangsmaß im oberen nicht sich verjüngenden Bereich mittels eines Formringes bestimmt. Aus einer Anzahl von vorgefertigten Formringen wird derjenige ausgewählt, der hinsichtlich seiner biomechanischen Form sowie seines Innenmantelumfanges am nächsten kommt. Der Formring ist spangenartig als nicht geschlossener Ring ausgebildet und besteht vorzugsweise aus einem additionsvernetzten, toxikologisch unbedenklichen Silikon-Kautschuk. Wie weiter unten noch beschrieben wird, reichen in der Regel drei unterschiedlich große Formringe I-III (siehe Fig. 9) aus, um die geforderte Maßgenauigkeit zu erhalten. Im vorliegenden Fall besitzt der Formring eine Höhe von etwa 15 cm. In dem darunter liegenden, sich verjüngenden Bereich des Oberschenkelstumpfes 12 werden jeweils im Abstand von 5 cm Umfangsmaße 4-6 genommen, die zusammen mit der Restkörperstumpflänge l₁₂ und der Formringgröße einem Rechner 17 eingegeben werden. Der Formring kann auch als geschlossener Ring ausgebildet sein.

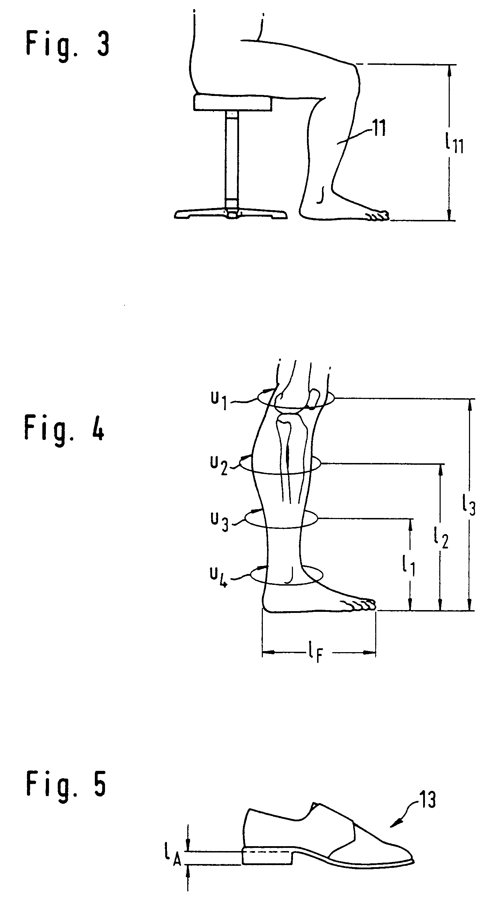

Für die Herstellung einer kosmetischen Verkleidung für die Prothese sind ferner die Unterschenkellänge l₁₁ sowie jeweiligen Umfangsmaße des Knies U₁, der Wade U₂ und des Knöchels U₄ des verbliebenen Beines maßgeblich. Zusätzlich kann im Übergangsbereich zwischen Wade und Knöchel ein weiterer Umfang U₃ gemessen werden. Neben der Unterschenkellänge l₁₁ sind die jeweiligen Längenmaße l₁, l₂ und l₃ vom verbliebenen Patientenbein zu nehmen, um auch insoweit die Prothesenverkleidung im Unterschenkelbereich anpassen zu können. Schließlich werden am verbliebenen Patientenbein noch die Fußlänge l_{f} (Schuhgröße) bestimmt, um den in Fig. 5 dargestellten Schuh 13 anfertigen zu können. Hierbei wird auch die gewünschte Absatzhöhe l_{A} berücksichtigt.

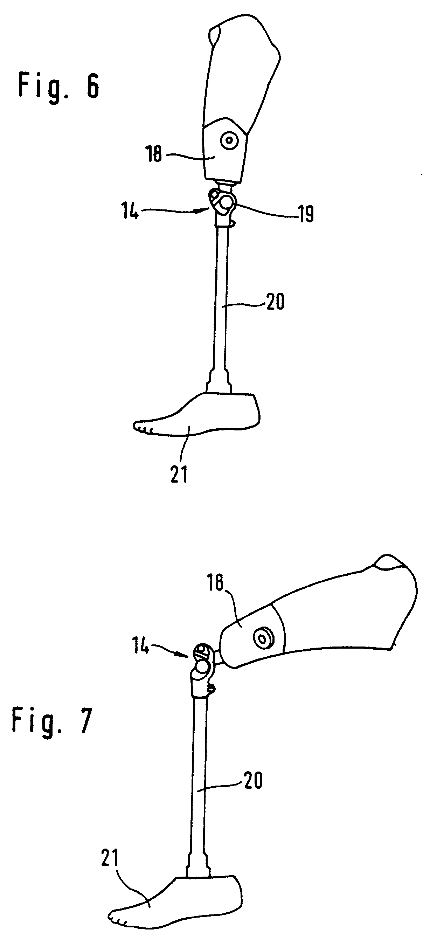

Die Fig. 6 und 7 stellen die gesamte Prothese 14 ohne Verkleidung dar.

Das künstliche Kniegelenk 19 besitzt einen Zapfen zur Befestigung an dem Oberschenkelschaft 18, wobei der Zapfen Teil eines Klemmkörpers ist, der in dem Kniegelenk 19 schwenkbar befestigt ist. Ferner ist das Unterschenkelrohr 20 mit einem endseitig befestigtem künstlichen Fuß 21 drehgesichert mit dem drehbaren, im Kniegelenk 19 gelagerten Walzenkörper verbunden.

In einer Ausführungsvariante ist im vorderen Bereich des Kniegelenkes im Kniegelenkgehäuse ein Walzenkörper um eine horizontale Drehachse gelagert, wobei die Stirnseiten des Walzenkörpers eine durchgehende zentrale Gewindebohrung zur Verschraubung des Walzenkörpers mit den Gabelarmen des Teils aufweisen, der zur Aufnahme des Unterschenkelrohres 20 dient. Jede der Stirnseiten des Walzenkörpers besitzt eine Parallelrippe, die in einen Schlitz jedes Gabelarmes einführbar ist. Die Führungswulst der Gabelarme dient zur Positionierung der Gabelarme gegenüber dem mit ihnen fest verbundenen Walzenkörper. Zur Befestigung dient eine Schraube mit möglichst breitflächigem Kopf. Im hinteren Bereich besitzt das Kniegelenk ein Gehäuse mit einer gabelförmigen Aufnahme für eine Schwenkachse des Klemmkörpers. Die Schwenkachse besteht aus einem in der gabelförmigen Aufnahme gelagerten Bolzen, der eine Bohrung des Klemmkörpers durchgreift. In dem Bereich, in dem der Klemmkörper und der Walzenkörper bei vertikaler Belastung schließlich zur Anlage kommen, ist in dem Klemmkörper ein Stahlteil in eine Öffnung eingesetzt, wobei das Stahl selbst eine abgeflachte und gehärtete Klemmfläche besitzt. Das Kniegelenk besitzt ferner eine Druckfeder, welche die Ausrastspannung des Kniegelenkes unterstützt. Über eine im Kniegelenkgehäuse aufgenommene Stellschraube kann der Klemmkörper in seiner Lage zwischen dem Walzenkörper und dem Stahlteil stufenlos justiert werden. Zusätzlich ist ein Befestigungszapfen für einen Streckzug im unteren Bereich des Kniegelenkgehäuses horizontal liegend befestigt.

Um im Stolperfall ein unkontrolliertes Einknicken des künstlichen Kniegelenkes zu verhindern, ist vorgesehen, im Kniegelenkgehäuse sowohl einen drehgesichert, mit einem Teil zur Aufnahme des Unterschenkelrohres einer Beinprothese verbundenen Walzenkörper drehbar als auch den Klemmkörper derart schwenkbar zu lagern, daß bei einer vertikalen Belastung des Klemmkörpers dieser auf den Walzenkörper derart zuschwenkt, daß der Klemmkörper und der Walzenkörper zu einer eine Flexion verhindernden, sperrenden Anlage kommen. Solche oder ähnliche Kniegelenkgehäuse sind nach dem Stand der Technik im Prinzip bekannt. Ein derart vorangehend beschriebenes Kniegelenk ist Gegenstand der europäischen Patentanmeldung 88112909.2, auf die Bezug genommen wird.

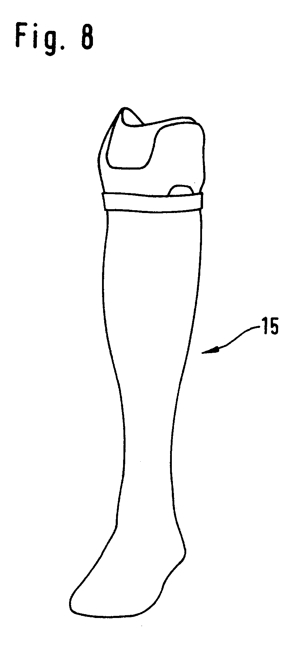

Fig. 8 zeigt eine kosmetische Verkleidung 15 für eine Unterschenkelprothese, etwa zum Einsatz bei einer Prothese nach den Fig. 6 und 7. Diese Verkleidung ist bezüglich ihrer Länge sowie der biomechanischen Form als auch den verschiedenen Umfangsmaßen im Knöchel-, Waden- und Kniebereich des verbliebenen Beines angepaßt.

Das erfindungsgemäße Verfahren ist schematisch in Fig. 9 dargestellt.

Bei der Herstellung eines positiven Restkörperteils nach Fig. 2 wird zunächst aus vorgefertigten Formringen I-III derjenige ausgewählt, der dem Umfangsmaß oberhalb der sich verjüngenden Stumpfkuppe am nächsten kommt. Der Formring 16 ist durch Umfang sowie seine biomechanische Form bestimmt. Nach Wahl des geeigneten Formringes, durch den das Stumpfoberteilmaß erhalten wird, erfolgt die Vermessung der Stumpfkuppe, in dem im Abstand von beispielsweise 5 cm jeweilige Umfangsmaße a, b und c genommen werden. Zusätzlich wird die Länge l₁₂ ermittelt.

Die ausgewählte Formringgröße, die Stumpfkuppenumfangsmaße und die Länge werden in den Rechner 17 gegeben. Der Speicher dieses Rechners kann anhand der Formringeingabe I, II oder III feststellen, welcher Formring zugrundegelegt werden soll. Der Rechner enthält weiterhin die Maße von mindestens zwei theoretischen Urformen A und B, und zwar in Form von Wertesets, etwa Zahlentripeln, Länge x, Polarkoordinaten y des Radius und Winkelmaß z. Jedes dieser Zahlentripel kennzeichnet eine Scheibe der Stumpfkuppe. Im vorliegenden Fall sind an dem Urmodell jeweils drei Scheiben angenommen, die jeweils in dem Bereich liegen, in dem die Umfangsmaße a, b und c sowie der Formring I-III liegen. Unterschiedliche Stumpfkuppen werden von der Urform dadurch abgeleitet, daß die Abstände der Scheiben im Rechner verändert werden. Mit anderen Worten, die Stumpfkuppen-Urform A oder B kann durch axiale Stauchung oder Strekkung variiert werden. Die Urformen A und B unterscheiden sich im wesentlichen durch ihre biomechanische Form, etwa nach der Einteilung schmal-breit. Der Rechner nimmt aufgrund der eingegebenen Werte I, II oder III einerseits sowie a, b oder c andererseits und der Länge l₁₂ die Klassifizierung der Stumpfkuppe nach Urform A oder B vor. Die aufgrund der Meßdaten eingegebenen Informationen werden dergestalt verarbeitet, daß der Rechner durch Vergleich mit den gegebenenfalls gestreckten oder gestauchten Urformen A oder B betreffende Daten der Stumpfkuppe zusammenstellt. Diese Daten werden in Form von Steuerimpulsen an eine Führungseinrichtung 22 für einen Fräser 23 gegeben, der einen Vollzylinder-Formkörper 24 bearbeitet. Steuerungsvorrichtungen für Fräser sind nach dem Stand der Technik bekannt. Aus dem Formkörper 24 wird ein positives Stumpfmodell gefräst, das zur Schaftherstellung dient. Der eigentliche Prothesenschaft aus Kunststoff wird aus dem Positiv durch Tiefziehen oder Gießen gewonnen.

Nach dem gleichen Prinzip wird auch die kosmetische Prothesenverkleidung hergestellt. Hierzu werden die in Fig. 3 und 4 dargestellten Längenmaße l₁₁, l₁, l₂, l₃ und l_{f} sowie betreffende Knie-, Waden-, Knöchel- und Zwischenbereichmaße U₁ bis U₄ genommen. Im Speicher des Rechners 17 sind ebenso wie bei der Stumpfkuppe mindestens zwei Urformen für die kosmetische Verkleidung mit ihren Werten und Maßen scheibenweise gespeichert. Die eingegebenen Längen- und Umfangsmaße des verbliebenen gesunden Patientenbeines werden im Rechner mit den entsprechenden Maßen der dort gespeicherten Urformen verglichen, gegebenenfalls nach Stauchung oder Streckung der Urformen. Die hieraus ermittelten Daten können entsprechend Fig. 9 als Steuerimpulse einer Steuervorrichtung 22 übertragen werden, welche einen Fräser 23 steuert, der aus einem zylinrischen Vollkörper die prothetische Verkleidung fräst. Wird der formkörper gedreht, wobei die Drehgeschwindigkeit einer entsprechenden Welle ebenfalls von dem Rechner bzw. der Steuervorrichtung geregelt wird, so benötigt der Fräser lediglich eine Führung in y-Richtung (Polarkoordinat des Radius) und in x-Richtung (Axialrichtung des Stumpfes bzw. der kosmetischen Verkleidung).

## Patentansprüche

1. Verfahren zur Herstellung einer Oberschenkelprothese, bei dem aus einem den Restkörperteilmaßen entsprechenden Positivmodell ein Prothesenschaft mit einem an diesem verankerten Gelenkpaßteil geformt und anschließend der Prothesenschaft mit einer kosmetischen Verkleidung überzogen oder umhüllt wird,
dadurch gekennzeichnet
daß von mindestens zwei theoretischen Urformen für Restkörperstümpfe und/oder kosmetische Verkleidungen deren jeweilige radiale Umfangslinien scheibenweise als Wertesets (Länge x, Polarkoordinaten y des Radius, Winkelmaß z) gespeichert werden, wobei am Patientenrestkörperstumpf jeweils der Umfang oberhalb der sich verjüngenden Stumpfkuppe und in vorgegebenen Abständen im Bereich der sich verjüngenden Stumpfkuppe und/oder der verbliebenen rechten oder linken Extremität (Bein) sowie die jeweilige Länge der Stumpfkuppe und/oder der Extremität gemessen und mit den gespeicherten Wertesets verglichen werden, wobei der Umfang im umfangsgleichen Bereich der Stumpfkuppe oberhalb deren konischen Bereichs durch Anpassung eines von mehreren, vorzugsweise von drei spangenartigen, nicht geschlossenen, eine Höhe von etwa 15 cm aufweisenden Formringen ermittelt wird, und daß die dem Restkörperstumpf oder der Extremität nächstkommende theoretische Urform ausgewählt und deren Wertesets zur Formung eines Positivmodells verwendet werden, wobei zur Herstellung der kosmetischen Verkleidung für die Prothese die Unterschenkellänge (l₁₁), sowie die Umfangsmaße des Knies (U₁), der Wade (U₂), des Knöchels (U₄) und eines zwischen der Wade (U₂) und dem Knöchel (U₄) liegenden Beinzwischenbereichs des verbliebenen Beines sowie deren Längen (l₁, l₂, l₃) von der Fußstandfläche zum Beinzwischenbereich, zur Wade und zum Knie als Werte eingespeichert, die als Steuerimpulse zur vollautomatischen Steuerung einer Führungseinrichtung für einen, einen Vollzylinder bearbeitenden Fräser verwendet werden.

2. Verfahren nach Anspruch 1,
dadurch gekennzeichnet,
daß die Formringe unterschiedliche biomechanische Formen besitzen.

3. Verfahren nach einem der Ansprüche 1 oder 2,
dadurch gekennzeichnet,
daß die durch die Wertesets bestimmten Scheiben der Urformen und/oder die vorgegebenen Abständen, in denen die Stumpfkuppe vermessen wird, äquidistant, vorzugsweise 5 cm sind.

4. Verfahren nach einem der Ansprüche 1 bis 3,
dadurch gekennzeichnet,
daß die kosmetische Verkleidung durch mindestens drei Wertesets der noch verbleibenden Extremität bestimmt wird.

5. Verfahren nach einem der Ansprüche 1 bis 4,
dadurch gekennzeichnet,
daß die Urformen durch Abstandsveränderung der Scheiben (Wertesets) in der Länge variiert werden, und aus den sich ergebenen Variationen die nächstkommende theoretische Urform ausgewählt wird.

6. Verfahren nach Anspruch 5,
dadurch gekennzeichnet,
daß die fehlenden, den jeweiligen Umfangsverlauf bestimmenden Werte zwischen den Scheiben durch Interpolation ermittelt werden.

7. Verfahren nach einem der Ansprüche 1 bis 6,
dadurch gekennzeichnet,
daß die Positivmodelle (Stumpfkuppe, kosmetische Verkleidung) durch Abtragen, insbesondere Drehen oder Fräsen aus einem Formkörper hergestellt werden.

8. Verfahren nach Anspruch 7,
dadurch gekennzeichnet,
daß der Formkörper aus Kunststoff, vorzugsweise Hartschaumkunststoff, besteht.

9. Verfahren nach einem der Ansprüche 1 bis 8,
dadurch gekennzeichnet,
daß der Formkörper scheibenweise bearbeitet wird.

10. Verfahren nach einem der Ansprüche 1 bis 9,
dadurch gekennzeichnet,
daß der Fräser von einem Rechner gesteuert wird, der gleichzeitig als Speicher und Komparator dient.

## Claims

1. Process for the production of an above-knee prosthesis, in which, from a positive model corresponding to the dimensions of the remaining body part, a prosthesis shank with an articulated fitting part anchored upon the same is formed and the prosthesis shank is afterwards coated or ensheathed with a cosmetic covering,
**characterized in that**,
of at least two theoretical original configurations for remaining body part Stumps and/or cosmetic coverings, their respective radial circumferential lines are stored in slices in the form of value sets (length x, polar coordinates y of the radius, angular measurement z), in which case, on the remaing stump of the patient's body part, in each case the circumference above the tapering stump cone and at predetermined distances within the area of the tapering stump cone and/or the remaining right or left extremity (leg) as well as the respective length of the stump cone and/or of the extremity are measured and compared with the stores value sets, in the course of which the circumference within the peripherally identical region of the stump cone above the conical area of the same, by means of adaptation of one of several, by preference three, bracelet-like, non-closed shaped rings possessing a height of approximately 15 cm, is determined and in that the theoretical original configuration coming nearest to the remaining body part stump or the extremity is selected and their value sets are employed for the moulding of a positive model, in which case, for the production of the cosmetic covering for the prosthesis, the length of the lower leg (l₁₁) as well as the circumferential dimensions of the knee (U₁), the calf (U₂), the ankle ((U₄) and of an intermediate leg region of the remaining leg as well as their lengths (l₁ , l₂, l₃) from the base of the the intermediate leg region, to the calf and to the knee are stored in the form of values, which are used as control pulses for the fully automatic control of a guide means for a milling cutter machining a solid cylinder.

2. Process according to Claim 9,
**characterized in that**
the shaped rings possess different biomechanical configurations.

3. Process according to either Claim 1 or 2,
**characterized in that**
the slices determined by the value sets of the original configurations and/or the predetermined distances at which the stump cone is measured, are equidistant, by preference 5 cm.

4. Process according to any of Claims 1 to 3,
**characterized in that**
the cosmetic covering is determined by at least three value sets of the still extant extremity.

5. Process according to any of Claims 1 to 4,
**characterized in that**
the original configurations are varied in length by a change in the distance between the slices (value sets) and in that, from the resulting variations, the theoretical configuration coming nearest is selected.

6. Process according to Claim 5,
**characterized in that**
the missing valued between the slices determining the respective course of the circumference are determined by interpolation.

7. Process according to any of Claims 1 to 6,
**characterized in that**
the positive models (stump cone, cosmetic covering) are produced by machining, in particular by turning or milling from a shaped member.

8. Process according to Claim 7,
**characterized in that**
the shaped member si comprised of plastic, preferably a hard foamed plastic.

9. Process according to any of Claims 1 to 8,
**characterized in that**
the shaped member is machined in slices.

10. Process according to any of Claims 1 to 9,
**characterized in that**
the milling cutter is controlled by a computer which at the same time serves as memory and comparator.

## Revendications

1. Procédé pour la fabrication d'une prothèse pour amputé fémoral dans lequel, à partir d'un modèle positif qui correspond aux dimensions du membre qui reste, une tige de prothèse est formée avec une pièce ajustée articulée, ancrée sur celle-ci, et ensuite la tige de prothèse est revêtue ou enveloppée par un habillage cosmétique,
caractérisé en ce
que les lignes circonférentielles radiales respectives d'au moins deux formes d'origine théoriques pour les moignons du membre et/ou les habillages cosmétiques sont mémorisées disque par disque comme sets de valeurs (longueur x, coordonées polaires y du rayon, mesure de l'angle z), la circonférence au-dessus du moignon qui s'effile en cône et à certains écarts prédéterminés dans la zone du moignon qui s'effile en cône et/ou de l'extrémité droite ou gauche qui reste (jambe) ainsi que la longueur respective du moignon et/ou de l'extrémité étant mesurées sur le moignon du patient et étant comparées aux sets de valeurs mémorisées, la circonférence dans la zone de même circonférence du moignon au-dessus de la zone conique de celui-ci étant déterminée par l'adaptation de l'un de plusieurs anneaux moulés, de préférence de trois anneaux moulés de type agrafe, non fermés, qui présentent une hauteur d'environ 15 cm et que la forme d'origine théorique qui s'approche le plus du moignon ou de l'extrémité étant sélectionnée et ses sets de valeurs étant utilisés pour façonner un modèle positif, la longueur de la jambe (l₁₁) ainsi que les dimensions de la circonférence du genou (U₁), du mollet (U₂), de la cheville (U₄) et d'une zone intermédiaire de la jambe située entre le mollet (U₂) et la cheville (U₄) du reste de la jambe, ainsi que leurs longueurs (l₁, l₂, l₃) de la surface de pose du pied jusqu'à la zone intermédiaire de la jambe, jusqu'au mollet et au genou étant mises en mémoire pour fabriquer l'habillage cosmétique pour la prothèse, valeurs qui sont utilisées comme impulsions de commande pour la commande entièrement automatique d'un dispositif de guidage pour une fraise qui usine un cylindre plein.

2. Procédé selon la revendication 1,
caractérisé en ce
que les anneaux moulés possèdent différentes formes biomécaniques.

3. Procédé selon l'une des revendications 1 ou 2,
caractérisé en ce
que les disques des formes d'origine, qui sont déterminés par les sets de valeurs, et/ou les écarts prédéterminés, auxquels le moignon est mesuré, sont équidistants, de préférence de 5 cm.

4. Procédé selon l'une des revendications 1 à 3,
caractérisé en ce
que l'habillage cosmétique est déterminé par au moins trois sets de valeurs de l'extrémité qui reste.

5. Procédé selon l'une des revendications 1 à 4,
caractérisé en ce
que les formes d'origine varient par la modification de l'écart entre les disques (sets de valeurs) dans la longueur et que la forme d'origine théorique la plus proche est sélectionnée parmi les variations qui résultent.

6. Procédé selon la revendication 5,
caractérisé en ce
que les valeurs qui manquent, qui déterminent le cours respectif de la circonférence, entre les disques sont déterminées par interpolation.

7. Procédé selon l'une des revendications 1 à 6,
caractérisé en ce
que les modèles positifs (moignon, habillage cosmétique) sont fabriqués par l'action d'enlever, en particulier par tournage ou fraisage d'un corps moulé.

8. Procédé selon la revendication 7,
caractérisé en ce
que le corps moulé est constitué en matière plastique, de préférence en mousse rigide plastique.

9. Procédé selon l'une des revendications 1 à 8,
caractérisé en ce
que le corps moulé est usiné disque par disque.

10. Procédé selon l'une des revendications 1 à 9,
caractérisé en ce
que la fraise est commandée par un ordinateur qui sert simultanément de mémoire et de comparateur.
